(19) Europäisches Patentamt
European Patent Office
Office européen des brevets

(11) Veröffentlichungsnummer: **0 136 566**
**B1**

# (12) EUROPÄISCHE PATENTSCHRIFT

(45) Veröffentlichungstag der Patentschrift: **01.04.87**

(51) Int. Cl.⁴: **C 07 C 25/02,** C 07 C 17/12

(21) Anmeldenummer: **84110415.1**

(22) Anmeldetag: **01.09.84**

## (54) Verfahren zur Herstellung von 3,4-Dichlorbenzotrihalogeniden.

(30) Priorität: **06.09.83 DE 3332017**

(43) Veröffentlichungstag der Anmeldung: **10.04.85 Patentblatt 85/15**

(45) Bekanntmachung des Hinweises auf die Patenterteilung: **01.04.87 Patentblatt 87/14**

(84) Benannte Vertragsstaaten: **BE CH DE FR GB IT LI**

(56) Entgegenhaltungen:
**DE - A - 2 520 815**
**DE - A - 2 644 641**

**Chemische Technologie, Band 6 (1982), 154-156**
**Ullmann, Band 3 (1973), 350 und Band 1 (1972), 227**

(73) Patentinhaber: **BASF Aktiengesellschaft, Carl-Bosch-Strasse 38, D-6700 Ludwigshafen (DE)**

(72) Erfinder: **Parg, Adolf, Dr., Paray-le-Monial-Strasse 8, D-6702 Bad Duerkheim (DE)**
Erfinder: **Husslein, Gerd, Dr., Herrenbergstrasse 2, D-6702 Bad Dürkheim (DE)**
Erfinder: **Hamprecht, Gerhard, Dr., Rote-Turm-Strasse 28, D-6940 Weinheim (DE)**

**EP 0 136 566 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von 3,4-Dichlorbenzotrihalogeniden, ausgehend von 4-Monochlorbenzotrihalogeniden.

In der Literatur sind bereits verschiedene Verfahren zur Herstellung von 3,4-Dichlorbenzotrihalogeniden beschrieben worden.

Gemäss GB-A-771 416 erhält man 3,4-Dichlorbenzotrichlorid durch ca. 8stündige Behandlung von 1,4-Bis(trichlormethyl)-2-chlorbenzol mit Chlor bei einer Temperatur von ca. 250°C. Ein wesentlicher Nachteil des Verfahrens besteht darin, dass das verwendete Ausgangsprodukt nur in einem mehrstufigen Syntheseprozess erhalten werden kann.

Weiterhin ist aus DE-A-2 333 848 bekannt, 3,4-Dichlorbenzotrifluorid durch Chlorierung von 4-Chlorbenzotrifluorid in Gegenwart von 10 Mol-% Eisen(III)chlorid herzustellen. Nachteilig ist hier einerseits die benötigte grosse Katalysatormenge, die eine einfache Aufarbeitung erschwert und zu Ausbeuteverlusten führt und andererseits die gleichzeitige Entstehung höher chlorierter Produkte (2,4,5- bzw. 3,4,5-Trichlorbenzotrifluorid), die mittels aufwendiger Destillation abgetrennt werden müssen.

Schliesslich wird in der DE-A-2 644 641 die Herstellung von 3,4-Dichlorbenzotrichlorid, ausgehend von 4-Chlorbenzotrichlorid und Chlor bzw. einem Chlor freisetzenden Mittel, in Gegenwart von Eisen(III)chlorid oder Aluminiumchlorid oder deren jeweiligen Gemischen mit Schwefeldichlorid oder Dischwefeldichlorid beschrieben.

Einer industriellen Produktion nach diesem Verfahren stehen allerdings folgende Nachteile entgegen:

a) Das Verfahren benötigt einen grossen Überschuss an Chlor (bis zu 20 Mol Chlor bzw. Chlor freisetzende Mittel pro Mol 4-Chlorbenzotrichlorid).
b) Gute Ausbeuten werden nur beim Einatz von Gemischen aus Aluminiumchlorid/Dischwefeldichlorid oder Eisen(III)chlorid/Schwefeldichlorid bzw. Dischwefeldichlorid als Katalysatoren erhalten.
c) Aufwendige Aufarbeitung durch Beseitigung der Katalysatorrückstände.
d) Schlechte Raum-Zeit-Ausbeute durch geringe Chlorierungsgeschwindigkeit.

Es wurde nun gefunden, dass man 3,4-Dichlorbenzotrihalogenide der Formel I

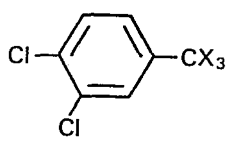
I,

in der X ein Fluor- oder Chloratom bedeutet, durch Umsetzung der entsprechenden4-Monochlorbenzotrihalogenide mit Chlorgas, in Gegenwart katalytischer Mengen einer Lewis-Säure vorteilhaft und ohne die eingangs erwähnten Nachteile herstellen kann, wenn man das Reaktionsgemisch in einer Umlaufapparatur mit statischen Mischern mittels einer Pumpe im Kreis führt und bei einer Temperatur von 20 bis 120°C die äquivalente Menge Chlorgas, bezogen auf das entsprechende 4-Monochlorbenzotrihalogenid, entsprechend seiner Umsetzung einleitet.

Als Lewis-Säuren bezeichnet man bekanntlich alle die Stoffe, die als Elektronenpaar-Akzeptor wirken. In der vorliegenden Erfindung sind darunter hauptsächlich diejenigen Lewis-Säuren zu verstehen, die üblicherweise als Katalysatoren bei der Chlorierung von Aromaten Anwendung finden (vgl. Houben-Weyl «Methoden der Organischen Chemie» 5/3, S. 652 f.), beispielsweise Eisen(III)chlorid, Aluminiumchlorid, Antimon(III)chlorid, Antimon(V)chlorid, Zinn(IV)chlorid. Als besonders vorteilhaft erweist sich die Verwendung von Eisen(III)chlorid.

Die Menge der eingesetzten Lewis-Säure liegt im Bereich von 1 bis 50 mMol, insbesondere 2 bis 10 mMol, bezogen auf 1 Mol 4-Monochlorbenzotrihalogenid.

Ihr Zusatz erfolgt vor der Umsetzung. Diese wird vorzugsweise lösungsmittelfrei durchgeführt. Gegebenenfalls können aber auch inerte Lösungsmittel wie chlorierte Kohlenwasserstoffe, beispielsweise 1,1,2,2- oder 1,1,1,2-Tetrachlorethan, Tetrachlorethylen, Tetrachlorkohlenstoff, 1,1,1- oder 1,1,2-Trichlorethan oder 1,2,4-Trichlorbenzol verwendet werden.

Da die Ausgangsprodukte 4-Monochlorbenzotrifluorid bzw. -chlorid im obengenannten Temperaturbereich in flüssiger Form vorliegen, erhält man als Reaktionsgemisch eine Suspension einerseits, für den Fall, dass man feste Lewis-Säuren ($FeCl_3$, $AlCl_3$) verwendet, und eine Emulsion andererseits, für den Fall, dass man flüssige Lewis-Säuren ($SbCl_5$, $SnCl_4$) verwendet.

Das Reaktionsgemisch, in das man Chlorgas einleitet, wird erfindungsgemäss in einer Umlaufapparatur im Kreis geführt. Dazu kann z.B. ein Kreislauf- oder Ringraumreaktor verwendet werden, der mit einer Pumpe, vorteilhaft mit einer Kreiselpumpe, und einer Heizquelle ausgerüstet ist.

Vor dem Einleiten des Chlorgases erhitzt man das Reaktionsgemisch auf die erforderliche Temperatur. Der geeignete Temperaturbereich liegt bei 20 bis 120°C, wobei der Bereich von 40 bis 90°C bevorzugt ist.

Beim Einleiten des Chlorgases in den Reaktor ist Sorge zu tragen, dass das Chlorgas mit dem Reaktionsgemisch gut durchmischt wird. Dies wird durch die Verwendung von statischen Mischern oder Mischelementen gewährleistet, die sich in der Umlaufapparatur befinden.

Erfindungsgemäss leitet man das Chlorgas entsprechend seiner Umsetzung in das Reaktionsgemisch ein. Dabei ist es von besonderem Vorteil, wenn das Chlorgas möglichst lange mit dem 4-Chlorbenzotrihalogenid/Lewis-Säure-Gemisch in Berührung kommt. Diese Erhöhung der Verweilzeit des Chlorgases im Reaktionsgemisch wird erfindungsgemäss dadurch erreicht, dass man das Reaktionsgemisch im Kreis führt.

Dies hat zur Folge, dass die benötigte Chlormenge auf die äquimolare Menge, bezogen auf das 4-Monochlorbenzotrihalogenid, beschränkt werden

kann. In manchen Fällen kann es auch von Vorteil sein, einen 10%igen Überschuss an Chlorgas einzuleiten.

Das Verfahren wird in diskontinuierlicher Arbeitsweise durchgeführt, lässt sich aber auch in kontinuierlicher Arbeitsweise ausüben.

Man arbeitet entweder unter atmosphärischem Druck oder unter erhöhtem Druck im Bereich von 2 bis 100 bar.

Die durchschnittlichen Reaktionszeiten können dabei innerhalb eines grossen Bereichs schwanken. Im allgemeinen betragen sie 0,5 bis 12 Stunden.

Zur Isolierung des Reaktionsprodukts versetzt man das Reaktionsgemisch nach beendeter Umsetzung mit verdünnter Salzsäure, trennt die organische Phase ab, wäscht sie mit Wasser und trocknet sie und unterwirft sie anschliessend der fraktionierten Destillation. Gegebenenfalls kann der Katalysator vor der Aufarbeitung durch einfache Filtration entfernt werden.

Mit dem erfindungsgemässen Verfahren werden die gewünschten 3,4-Dichlorbenzotrihalogenide isomerenfrei in einer Ausbeute $\geq$ 90% und in einer Reinheit von 97% erhalten.

Durch die Anpassung der Zufuhr des Chlorgases an seine Aufnahme im Reaktionsgemisch wird beim erfindungsgemässen Verfahren die Reaktionszeit gegenüber den bekannten Verfahren um mehr als die Hälfte verkürzt. Dies wird unterstützt durch eine Erhöhung der Verweilzeit des Chlorgases im Reaktionsgemisch. Durch die vollständige Umsetzung des zugesetzten Chlors in der Reaktionsmischung wird der Endpunkt der Umsetzung genau erfasst und ein Verlust von Chlor vermieden. Zur Feststellung des Endes der Umsetzung kann der Chlorgehalt des Abgases herangezogen werden, weil der Chlorgehalt im Abgas beim Ende der Umsetzung stark ansteigt.

Eine Umweltbelastung durch das bei der Umsetzung entstehende Abgas Chlorwasserstoff kann daher durch einfache Massnahmen vermieden werden, beispielsweise durch Absorption des Abgases in Wasser, wobei wiederverwendbare Salzsäure entsteht.

Ferner lässt sich im Vergleich zu den bekannten Verfahren die Katalysatormenge, insbesondere bei Verwendung von Eisen(III)chlorid, deutlich erniedrigen. Dadurch wird der Katalysator (Lewis-Säure) im Reaktionsgemisch wesentlich besser verteilt und kann nach Beendigung der Reaktion leichter abgetrennt werden, wobei nur minimale Ausbeuteverluste en Wertprodukt entstehen.

Die mit dem erfindungsgemässen Verfahren herstellbaren 3,4-Dichlorbenzotrihalogenide sind wichtige Zwischenprodukte zur Herstellung von substituierten Diphenylethern, die als Herbizide verwendet werden können.

Die folgenden Beispiele sollen die Erfindung näher erläutern.

*Beispiel 1*

Man verwendete eine Umlaufapparatur (s. Zeichnung) mit statischen Mischern, einer Kreiselpumpe, einer Heizquelle, einer Zuführung für Chlorgas sowie einer Abgasleitung für Chlorwasserstoff.

In dieser Apparatur wurde ein Gemisch aus 1 725 g (7,5 Mol) 4-Chlorbenzotrichlorid und 12 g (0,075 Mol) $FeCl_3$ auf eine Temperatur von 70°C erhitzt und dabei im Kreis geführt. Dann wurde mit der Einleitung von 585 g (8,25 Mol) Chlorgas begonnen. (Die Zufuhr des Chlorgases erfolgte mit solcher Geschwindigkeit, dass im Abgas kein Chlor feststellbar war.) Nach 5 Stunden war die Reaktion beendet. Das Reaktionsgemisch wurde mit 2 l verdünnter Salzsäure versetzt. Die organische Phase wurde abgetrennt, zweimal mit 2 l Wasser gewaschen und über $CaCl_2$ getrocknet. Durch anschliessende fraktionierte Destillation erhielt man 1 824 g 3,4-Dichlorbenzotrichlorid (92% d.Th.) (Reinheit 98% - GC) vom Siedepunkt 138 bis 140°C bei 12 mbar.

*Beispiel 2*

Man verwendete die in Beispiel 1 beschriebene Apparatur, in der ein Gemisch aus 1 470 g (9,67 Mol) 4-Chlorbenzotrifluorid und 3,2 g (0,02 Mol) $FeCl_3$ auf eine Temperatur von 60°C erhitzt und dabei im Kreis geführt wurde. Dann wurde analog Beispiel 1 mit der Einleitung von 754,3 g (10,63 Mol) Chlorgas begonnen. Nach 6 Stunden war die Reaktion beendet. Die Aufarbeitung erfolgte analog Beispiel 1. Man erhielt 1 996 g 3,4-Dichlorbenzotrifluorid (96% d.Th.) vom Siedepunkt 170 bis 172°C bei atmosphärischem Druck.

**Patentansprüche**

1. Verfahren zur Herstellung von 3,4-Dichlorbenzotrihalogeniden der Formel I

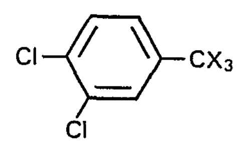

I,

in der X ein Fluor- oder Chloratom bedeutet, durch Umsetzung der entsprechenden 4-Monochlorbenzotrihalogenide mit Chlorgas, in Gegenwart katalytischer Mengen einer Lewis-Säure, dadurch gekennzeichnet, dass man das Reaktionsgemisch in einer Umlaufapparatur mit statischen Mischern mittels einer Pumpe im Kreis führt und bei einer Temperatur von 20 bis 120°C die äquimolare Menge Chlorgas, bezogen auf das entsprechende 4-Monochlorbenzotrihalogenid, entsprechend seiner Umsetzung einleitet.

2. Verfahren gemäss Anspruch 1, dadurch gekennzeichnet, dass man die Umsetzung bei einer Temperatur von 40 bis 90°C durchführt.

3. Verfahren gemäss Anspruch 1, dadurch gekennzeichnet, dass man die Umsetzung lösungsmittelfrei durchführt.

4. Verfahren gemäss Anspruch 1, dadurch gekennzeichnet, dass man als Lewis-Säure Eisen(III)-chlorid verwendet.

**Claims**

1. A process for the preparation of a 3,4-dichlorobenzotrihalide of the formula I

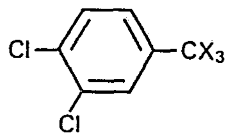

I,

where X is fluorine or chlorine, by reacting the corresponding 4-monochlorobenzotrihalide with chlorine gas in the presence of a catalytic amount of a Lewis acid, wherein the reaction mixture is recycled by means of a pump in a loop reactor with static mixers, and the amount of chlorine equivalent to the conversion of the corresponding 4-monochlorobenzotrihalide is introduced at 20-120°C.

2. A process as claimed in Claim 1, wherein the reaction is carried out at from 40 to 90°C.

3. A process as claimed in claim 1, wherein the reaction is carried out in the absence of a solvent.

4. A process as claimed in claim 1, wherein iron(III) chloride is used as the Lewis acid.

**Revendications**

1. Propcédé de préparation de trihalogénures de 3,4-dichlorobenzène de formule I

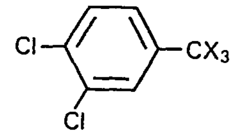

I,

dans laquelle X représente un atome de fluor ou de chlore, par réaction des trihalogénures de 4-monochlorobenzène correspondants avec le chlore gazeux, en présence de quantités catalytiques d'un acide de Lewis, caractérisé en ce qu'on fait circuler le mélange réactionnel, au moyen d'une pompe, dans un appareil travaillant en circuit fermé équipé de mélangeurs statiques et en ce qu'on y envoie, à une température de 20 à 120°C, la quanité équimolaire de chlore gazeux, par rapport au trihalogénure de 4-monochlorobenzène concerné, dans une mesure correspondant à sa transformation.

2. Procédé selon la revendication 1, caractérisé en ce qu'on mène la réaction à une température de 40 à 90°C.

3. Procédé selon la revendication 1, caractérisé en ce qu'on mène la réaction en l'absence de solvant.

4. Procédé selon la revendication 1, caractérisé en ce qu'on utilise, comme acide de Lewis, le chlorure ferrique.

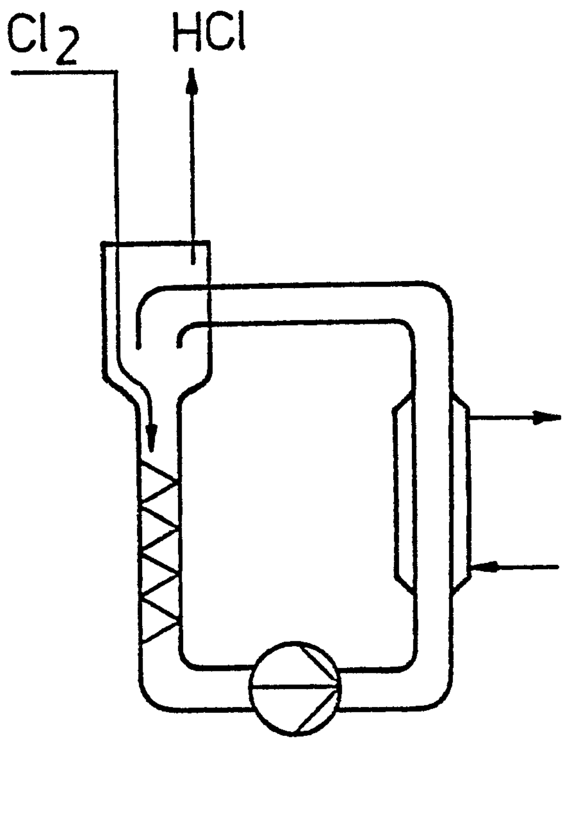
$Cl_2$
HCl